(19) Europäisches Patentamt
European Patent Office
Office européen des brevets

(11) Veröffentlichungsnummer: **0 029 890**
**B1**

(12) # EUROPÄISCHE PATENTSCHRIFT

(45) Veröffentlichungstag der Patentschrift: **15.09.82**

(51) Int. Cl.³: **C 07 C 102/00**, C 07 C 103/183

(21) Anmeldenummer: **80105811.6**

(22) Anmeldetag: **25.09.80**

(54) **Verfahren zur Herstellung von 4-Aminobuttersäureamid-hydrochlorid.**

(30) Priorität: **28.11.79 DE 2947825**

(43) Veröffentlichungstag der Anmeldung: **10.06.81 Patentblatt 81/23**

(45) Bekanntmachung des Hinweises auf die Patenterteilung: **15.09.82 Patentblatt 82/37**

(84) Benannte Vertragsstaaten: **BE CH FR GB IT LI NL SE**

(56) Entgegenhaltungen:
**ANGEWANDTE CHEMIE, Band 92, Nr. 8, 1980, Weinheim A. KLEEMANN et al. »Ein neuer Weg zu 4-Aminobuttersäureamid« Seite 640**
**CHEMISCHE BERICHTE, Band 92, Nr. 10, 1959, Weinheim C. BERTHER »Einseitige Verseifung von Dinitrilen und Hydrierung der entstandenen W-Cyan-Säureamide« Seiten 2616 bis 2621**
**H. O. HOUSE »Modern Synthetic Reactions« 1965, BENJAMIN, INC., New York, Amsterdam**

(73) Patentinhaber: **Degussa Aktiengesellschaft, Weissfrauenstrasse 9, D-6000 Frankfurt am Main 1 (DE)**

(72) Erfinder: **Kleemann, Axel, Dr. Dipl.-Chem., Greifenhagenstrasse 9, D-6450 Hanau 9 (DE)**
Erfinder: **Martens, Jürgen, Dr. Dipl.-Chem., Hochstrasse 5, D-8755 Alzenau (DE)**
Erfinder: **Weigel, Horst, Odenwaldstrasse 56, D-6458 Rodenbach (DE)**

**EP 0 029 890 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von 4-Aminobuttersäureamid-hydrochlorid

4-Aminobuttersäureamid und Derivate desselben haben Bedeutung als Therapeutika, insbesondere als Neurotransmitter.

Gegenstand der Erfindung ist ein Verfahren zur Herstellung von 4-Aminobuttersäureamid-hydrochlorid, welches dadurch gekennzeichnet ist, daß man 3-Cyanopropionsäureamid in Gegenwart eines unter den Reaktionsbedingungen inerten Lösungsmittels, eines Edelmetallkatalysators und von Chlorwasserstoff bei einer Temperatur zwischen 5 und 80° C hydriert.

Das erfindungsgemäße Verfahren ermöglicht es, auf sehr einfache Weise das Monohydrochlorid des 4-Aminobuttersäureamids herzustellen, das dann auf ebenfalls sehr einfache Weise, z. B. durch Behandlung mit einem basischen Ionenaustauscher oder mit einer geeigneten Base, in das 4-Aminobuttersäureamid umgewandelt werden kann.

Das als Ausgangsmaterial dienende 3-Cyanopropionsäureamid kann beispielsweise durch partielle Verseifung von Bernsteinsäuredinitril hergestellt werden. Seine Hydrierung erfolgt in Gegenwart eines unter den Bedingungen der Hydrierungsreaktion inerten Lösungsmittels. Geeignete Lösungsmittel sind ein- oder zweiwertige Alkohole mit 2 bis 6, vorzugsweise 2 bis 3, Kohlenstoffatomen oder deren Gemische mit Wasser, sowie Gemische von cyclischen Ethern mit 4 bis 10 Kohlenstoffatomen, insbesondere Tetrahydrofuran oder 1,4-Dioxan, mit Wasser. Die angewandte Menge an Lösungsmittel ist nicht kritisch, sie sollte aber zweckmäßigerweise so bemessen werden, daß das eingesetzte 3-Cyanopropionsäureamid bei der gewählten Reaktionstemperatur vollständig gelöst ist. Besonders bevorzugte Lösungsmittel sind geradkettige oder verzweigte Alkanole mit 2 bis 6 Kohlenstoffatomen, insbesondere Ethanol.

Die Hydrierung erfordert ferner die Anwesenheit eines Edelmetallkatalysators, insbesondere eines Platinmetallkatalysators. Besonders bevorzugte Katalysatoren sind metallisches Rhodium oder Platin und Platin-IV-oxid. Ebensogut können auch Gemische von mehreren Edelmetallen oder Gemische von Edelmetallen mit Platin-IV-oxid eingesetzt werden. Die Katalysatoren können in freier Form oder als Trägerkatalysatoren (z. B. auf Aktivkohle niedergeschlagen) angewandt werden. Sie können nach beendeter Hydrierung wiedergewonnen und ohne weitere Reinigung wiederholt eingesetzt werden, wobei es im Falle des Platin-IV-oxids unerheblich ist, ob dieses nach dem ersten Gebrauch partiell oder vollständig zu $Pt^{2+}$-Verbindungen oder metallischem Platin reduziert vorliegt. Die einzusetzende Menge an Edelmetallkatalysator ist nicht kritisch. Zur Erzielung kurzer Hydrierungszeiten empfiehlt es sich jedoch, den Edelmetallkatalysator in einer Menge von 1 bis 20, vorzugsweise von 5 bis 10 Gewichtsprozent, bezogen auf eingesetztes 3-Cyanopropionsäureamid, einzusetzen.

Die Hydrierung des 3-Cyanopropionsäureamids erfolgt schließlich in Gegenwart von Chlorwasserstoff, der zweckmäßigerweise in äquimolarer Menge zu dem eingesetzten 3-Cyanopropionsäureamid angewandt wird. Aber auch die Verwendung eines geringen Überschusses an Chlorwasserstoff ist möglich.

Die Hydrierung erfolgt bei einer Temperatur zwischen 5 und 80° C, vorzugsweise zwischen 25 und 50° C. Sie kann drucklos vorgenommen werden, indem man Wasserstoff durch das Reaktionsgemisch hindurchleitet, oder in einem druckfesten Reaktionsgefäß unter einem Wasserstoffdruck bis zu 100 bar. Vorzugsweise erfolgt die Hydrierung bei Drucken bis zu 5 bar. Der Wasserstoffdruck hat zwar einen gewissen Einfluß auf die für die vollständige Hydrierung erforderliche Zeit, die mit zunehmendem Druck etwas verkürzt wird, aber kaum auf die Reinheit des gebildeten 4-Aminobuttersäureamidhydrochlorids.

Nimmt man die Hydrierung in einem geradkettigen oder verzweigten Alkanol mit 2 bis 6, vorzugsweise 2 bis 3 Kohlenstoffatomen vor, so scheidet sich das gebildete 4-Aminobuttersäureamid-hydrochlorid während der Hydrierung oder, wenn diese bei höheren Temperaturen vorgenommen wird, nach dem Abkühlen des Reaktionsgemisches auf Raumtemperatur in kristalliner Form aus. Es kann dann zusammen mit dem Katalysator abfiltriert werden. Durch Digerieren des Filterkuchens mit warmem Methanol kann dann das 4-Aminobuttersäureamid-hydrochlorid in Lösung gebracht und vom Katalysator durch Dekantation oder Filtration abgetrennt werden.

Durch die nachfolgenden Beispiele soll die Erfindung näher erläutert werden:

## Beispiel 1

Durch ein Reaktionsgemisch aus 9,8 g (0,1 Mol) 3-Cyanopropionsäureamid, 3,65 g (0,1 Mol) Chlorwasserstoff, 0,75 g Platin-IV-oxid und 150 ml Ethylalkohol leitet man bei 30 bis 35° C unter Normaldruck Wasserstoffgas. Nach beendeter Wasserstoffaufnahme wird das auskristallisierte 4-Aminobuttersäureamid-hydrochlorid zusammen mit dem Katalysator abfiltriert. Der Filterkuchen wird mit warmem Methanol behandelt, wobei das 4-Aminobuttersäureamid-hydrochlorid in Lösung geht. Diese methanolische Lösung wird abfiltriert, aufkonzentriert und abgekühlt, wobei das 4-Aminobuttersäureamid-hydrochlorid erneut auskristallisiert.

Ausbeute: 11,5 g (83% der Theorie). Schmelzpunkt: 134—137° C.

$^1H-NMR(DMSO-d_6 + CDCl_3)$:

$\delta$ s 8,25 ($\gamma$ – $NH_3$, 3 H)

s 7,55 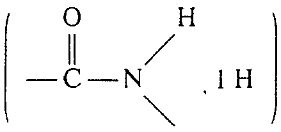 , 1 H

s 6,83 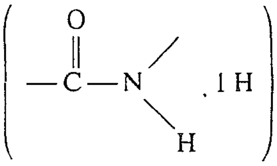 , 1 H

m 2,83 ($CH_2$, 2 H), m 2,22 ($CH_2$, 2 H)

und

m 1,9 ($CH_2$, 2 H)ppm .

Beispiel 2

Das Beispiel 1 wird wiederholt mit dem einzigen Unterschied, daß anstelle des Platin-IV-oxids 0,75 g metallisches Platin als Katalysator eingesetzt werden.

Ausbeute: 60% der Theorie. Schmelzpunkt: 134—136° C.

Beispiel 3

Das Beispiel 1 wird wiederholt mit dem einzigen Unterschied, daß anstelle des Ethanols 150 ml Isopropylalkohol als Lösungsmittel eingesetzt werden.

Ausbeute: 38% der Theorie. Schmelzpunkt: 134—136° C.

Beispiel 4

Das Beispiel 1 wird wiederholt mit dem einzigen Unterschied, daß anstelle des Ethanols 150 ml n-Butanol als Lösungsmittel eingesetzt werden.

Ausbeute: 39% der Theorie. Schmelzpunkt: 134—136° C.

Beispiel 5

Das Beispiel 1 wird wiederholt mit dem einzigen Unterschied, daß die Hydrierung in einem Schüttelautoklaven bei einem Wasserstoffdruck von 5 bar vorgenommen wird.

Ausbeute: 84% der Theorie. Schmelzpunkt: 135—137° C.

Beispiel 6

Das Beispiel 1 wird wiederholt mit dem einzigen Unterschied, daß die Hydrierung in Gegenwart von 0,8 g Rhodium anstelle des Platin-IV-oxids durchgeführt wird.

Ausbeute: 63% der Theorie. Schmelzpunkt: 136—138° C.

## Patentansprüche

1. Verfahren zur Herstellung von 4-Aminobuttersäureamidhydrochlorid, dadurch gekennzeichnet, daß man 3-Cyanopropionsäureamid in Gegenwart eines unter den Reaktionsbedingungen inerten Lösungsmittels, eines Edelmetallkatalysators und von Chlorwasserstoff bei einer Temperatur zwischen 5 und 80° C hydriert.
2. Verfahren nach Anspruch 1, dadurch gekennzeichnet, daß man als inertes Lösungsmittel ein geradkettiges oder verzweigtes Alkanol mit 2 bis 6 Kohlenstoffatomen einsetzt.
3. Verfahren nach einem der Ansprüche 1 oder 2, dadurch gekennzeichnet, daß man die Hydrierung bei einem Druck zwischen Normaldruck und 100 bar durchführt.
4. Verfahren nach einem der Ansprüche 1 bis 3, dadurch gekennzeichnet, daß man als Edelmetallkatalysator metallisches Platin und/oder Platin-IV-oxid einsetzt.

## Claims

1. A process for the production of 4-aminobutyric acid amide hydrochloride, characterised in that 3-cyanopropionic acid amide is hydrogenated in the presence of a solvent which is inert under the reaction conditions, and in the presence of a noble metal catalyst and hydrogen chloride at a temperature of from 5 to 80° C.
2. A process according to claim 1, characterised in that a straight-chain or branched alkanol having from 2 to 6 carbon atoms is used as the inert solvent.
3. A process according to one of claims 1 or 2, characterised in that hydrogenation is carried out at a pressure of from normal pressure to 100 bars.
4. A process according to one of claims 1 to 3, characterised in that metallic platinum and/or platinum-IV-oxide is used as the noble metal catalyst.

## Revendications

1. Procédé de fabrication de chlorhydrate de 4-aminobutyramide caractérisé en ce qu'on effecture une hydrogénation à une température comprise entre 5 et 80° C de 3-cyanopropionamide en présence d'un solvant inerte dans les conditions de réaction, d'un catalyseur à base de métal précieux et de chlorure d'hydrogène.
2. Procédé suivant la revendication 1 caractérisé en ce qu'on utilise à titre de solvant inerte un alkanol linéaire ou ramifié de 2 à 6 atomes de carbone.
3. Procédé suivant l'une des revendications 1 ou 2 caractérisé en ce qu'on effectue l'hydrogénation à une pression comprise entre la pression normale et 100 bars.
4. Procédé suivant l'une des revendications 1 à 3 caractérisé en ce qu'on utilise du platine métallique et/ou de l'oxyde de platine IV à titre de catalyseur à base de métal précieux.